# EUROPEAN PATENT SPECIFICATION

(11) **EP 1 877 771 B1**
(45) Date of publication and mention of the grant of the patent: **15.02.2012**
(21) Application number: 06732717.1
(22) Date of filing: 28.03.2006
(51) Int. Cl.: G01N 31/22

(54) **TUMOR SCREENING SYSTEM**
TUMOR-SCREENING-SYSTEM
SYSTÈME DE DÉPISTAGE TUMORA

(30) Priority: 28.03.2005 KR 20050025668; 28.03.2005 KR 20050025667; 28.03.2005 KR 20050025666; 18.04.2005 KR 20050032078
(43) Date of publication of application: 16.01.2008
(60) Divisional application: 11171195.8
(73) Proprietor: Medimex Co., Ltd., Jungwon-gu Seongnam-city, Kyunggi-do 462-729 (KR)
(72) Inventor: PARK, Chang Soo, Kwangju 501-843 (KR); MOON, Sang Ho, Kwangju 503-844 (KR)
(74) Representative: Pulieri, Gianluca Antonio
(86) International application number: PCT/KR2006/001124
(87) International publication number: WO 2006/104333

(56) References cited:
- US-A- 3 670 072
- US-A- 5 191 899
- US-A- 5 256 571
- US-A- 5 330 916
- US-A- 5 330 916
- US-A- 5 811 099
- US-A1- 2002 094 577
- US-B1- 6 327 377

## Description

### [Technical Field]

The present invention relates to a tumor screening system. The tumor screening system operates separation of cells from a vial containing a solution mixed with the cells and collection of the cells on a slide using an automatic unit.

### [Background Art]

The tumor screening system of the present invention operates separation of cells from a vial containing a solution mixed with the cells and collection of the cells on a slide using an automatic unit. A collection vial serves to simultaneously store a solution containing cells and pour the solution out of the tumor screening system.

Particularly, a collection vial is used to collect cervical cells and mix the collected cells with a solution for testing the cells.

Hereinafter, the collection vials for preserving cells in a solution and testing the cells are referred to as a solution vial.

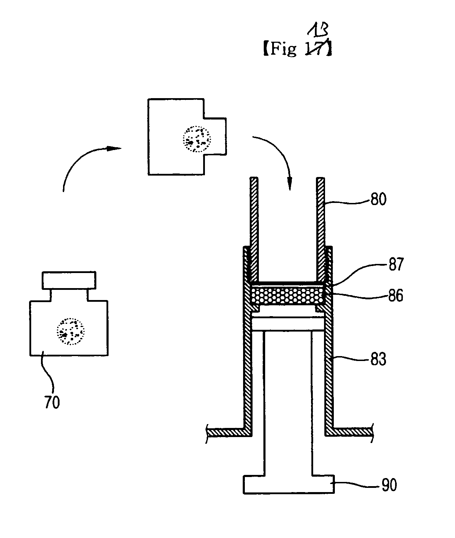

FIG. 13 is a schematic view partially illustrating a conventional solution vial and a conventional tumor screening system. As shown in FIG. 13, the conventional solution vial 70 contains collected cells, and is closed with a lid. The conventional solution vial 70 is shaken by user s hand or using other devices so that the cells are mixed with a solution in the solution vial 70. The solution vial 70 serves only to store the solution. Accordingly, the conventional tumor screening system comprises a separate container 80 for receiving the solution. That is, the conventional tumor screening system requires the container 80 in addition to the solution vial 70.

Since the container 80 of the conventional tumor screening system has an opened upper end surface, the solution in the container 80 contacts air. A cylindrical main body 83 supports a filter 86 provided with a collection film 87 mounted thereon, and the solution is sucked from the container 80 to the conventional tumor screening system by a piston 90 moving into and out of the main body 83. The main body 83 and the container 80 are connected by screws.

The solution vial for dispersing cells, stuck to a brush, into a solution is installed separately from the conventional tumor screening system. The solution in the solution vial under the condition that the cells are sufficiently dispersed into the solution by shaking the solution vial is poured into the container, a suction device contacting the lower surface of the filter sucks the solution, and then the cells filtered out with the filter are stuck to a slide.

When the container is separated from the main body so as to move the collection film close to the slide, the mixed solution remaining in the container pours out due to gravity, thus not being reused.

Further, since the transfer of the cells from the solution vial to the slide is performed by user's hands, it takes a long time to transfer the cells. Also, contaminants or impurities may be transferred from the user's hands to the slide, thus lowering the accuracy of cytological tests.

After the solution is poured into the container and a desired amount of the solution in the container is sucked by the suction device, the remainder of the solution in the container is already exposed to the air. Accordingly, when the remainder of the solution is reused, it is impossible to assure the accuracy of the tests.

When the filter is separated from the lower portion of the container, the remainder of the solution in the container pours out, and contaminates the system, thus causing sanitary problems.

Most of women's diseases relate to women's genital organs. i.e., uteri.

One of most common women's diseases is cervix carcinoma, which is a kind of tumor. Cervix carcinoma is a tumor having comparatively well-known pathogenesis, and is generated by a Human Papilloma Virus (HPV), which is a kind of venereal disease. In sexual intercourse, when woman's epithelial cells are infected by the HPV, DNA of the HPV penetrates DNA of cell nuclei, propagates, and generates a cancer of the epithelial cells.

Various methods for testing whether or not cells are infected by the HPV, including a cervix cytodiagnosis smear method, have been proposed. Recently, a Polymerase Chain Reaction (PCR) method is used to test whether or not cells are infected by the HPV. The PCR method is the most sensitive and accurate one of screening methods for early diagnosis of cervix carcinoma.

In order to use the PCR method, the collection of cells of a woman's uterus, particularly cells of a women's cervix, is required. Brushes having various shapes for collecting cells from a woman's uterus have been proposed.

Korean Utility Model Laid-open Publication No. 1999-1000001 (hereinafter, referred to as "reference 1") discloses 'cell collecting structure for diagnosing cervix disease'. The reference 1 discloses 'brush for collecting cervical cells', which is inserted into a woman's vagina and collects the cervical cells.

The 'cell collecting structure for diagnosing cervix disease' disclosed in reference 1 comprises an external membrane collecting unit and an internal membrane collecting unit detachably, which are connected to both sides of a handle, thereby causing inconvenience of separately collecting cells from the external membrane and the internal membrane of the cervix.

Further, Korean Patent Laid-open Publication No. 2004-82781 (hereinafter, referred to as "reference 2") discloses 'apparatus for collecting cervical cells'. The 'apparatus for collecting cervical cells' disclosed in reference 2 comprises a main collector made of silicon, a subsidiary collector, and a joint having an adjustable length.

The 'apparatus for collecting cervical cells' disclosed in reference 2 is advantageous in that the length of the apparatus is adjustable, but is disadvantageous in that the collection of the cells depends only on a collecting brush prepared at a first collecting unit and thus a cell collecting efficiency is not assured. Further, a second collecting unit is extended from the first collecting unit in a direction perpendicularly to the lengthwise direction of the first collecting unit, and stimulates the inner wall of a woman's vagina when the 'apparatus for collecting cervical cells' is inserted into the woman's vagina, thereby causing pain to a patient or an injury to the inner wall of the vagina.

In order to use the PCR method, collected cells are generally stored in a container filled with a cell fixing solution. In the structure and apparatus disclosed in references 1 and 2, since collecting units are directly separated from the brushes by operator's hands or using other tools, such as clamps, the collecting units may be contaminated with foreign substances. This obstructs the accurate diagnosis of the collected cells.

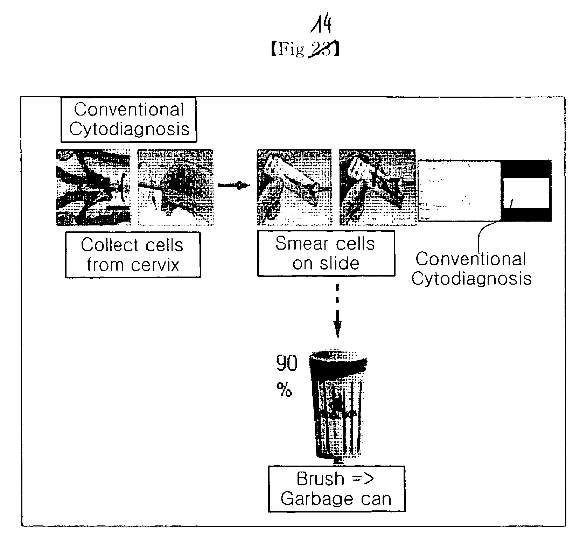

FIG. 14 is a schematic view illustrating conventional cytodiagnosis. With reference to FIG. 14, the conventional cytodiagnosis applied a pap smear method, in which cells are smeared on a slide and are then tested. When the cells are smeared on the slide, the cells on the slide are easily dried and a viscous solution and erythrocyte are not removed from the cells, thereby causing a difficulty in reading diagnosis results. Further, since only 10% of cells collected from a woman's cervix using a brush are smeared on the slide and the remainder of the collected cells are thrown away, cells, which are important to the diagnosis, may not be smeared on the slide. Accordingly, the conventional cytodiagnosis has a low reliability.

In order to perform the above liquid based cytology, a solution for preserving collected cells is required. Conventionally, the liquid based cytology requires 95% ethanol. The 95% ethanol cannot preserve cells for a long period of time (can preserve cells for approximately 1 week), and cannot remove erythrocyte from the cells, thus requiring a separate process for removing the erythrocyte from the cells. Further, nuclei of the cells preserved in the 95% ethanol are deformed and thus it is impossible to extract DNA from the cells. Moreover, the 95% ethanol has a high alcohol content and is inflammable, thus causing a danger of fire.

In order to solve the above problems, U. S. Patent Serial No. 5,256,571 discloses a cell preservative solution comprising a water-miscible alcohol, an anti-clumping agent, and a buffer agent. In the cell preservative solution, the alcohol is one selected from the group consisting of ethanol, isopropanol, and methanol, the anti-clumping agent is one selected from the group consisting of ethylenediamine tetraacetic acid and salts thereof, and the buffer agent is one selected from the group consisting of ethylenediamine tetraacetic acid, ethylenediamine tetraacetic acid salts, citric acid, and citric acid salts. However, since the amount of the alcohol for fixing the cells is 45~55 parts by weight, preferably 50 parts by weight, the cell preservative solution disclosed in U. S. Patent Serial No. 5,256,571 is still inflammable. Further, the cell preservative solution has a low erythrocyte removal rate, preserves the cells only for a comparatively short period of time (approximately 3 months), and causes a difficulty in extracting DNA from the cells.

### [Technical Problem]

Therefore, the present invention has been made in view of the above problems, and it is an object of the present invention to provide a tumor screening system, which operates separation of cells from a vial containing a solution mixed with the cells, and collection of the cells on a slide using an automatic unit.

### [Technical Solution]

In accordance with an aspect of the present invention, the above and other objects can be accomplished by the provision of a tumor screening system comprising solution vial operating units, each comprising a fixing unit for fixing a solution vial having a piston installed therein, and a cylinder device for linearly moving the solution vial toward a filter connected to the solution vial; piston operating units, each comprising a cylinder fixed to the corresponding solution vial operating unit, and a cylinder rod installed on the cylinder and fixed to one portion of the piston of the solution vial, for linearly moving piston; and suction units, each comprising a connecting portion disposed under the corresponding solution vial operating unit and connected to the filter, and a tube connected to the connecting portion for guiding a solution filling the solution vial, and a suction device connected to the inner space of the tube.

The tumor screening system further comprises slide operating units, each comprising a slide mounting portion on which a slide is mounted, and a moving device for moving the slide mounting portion to achieve contact between the slide and the filter connected to the corresponding suction unit.

Preferably, the tumor screening system may further comprise alcohol receiving units for receiving alcohol, and each of the slide operating units may further comprise a slide dropping unit for dropping the slide into the corresponding alcohol receiving unit.

Preferably, the tumor screening system may further comprise filter operating units, each comprising a moving unit for moving the filter to a position under the solution vial, and a fixing unit for fixing the filter.

Preferably, the tumor screening system may further comprise mixed solution forming units, each comprising a connecting portion, to which the solution vial is connected, and a rotating portion for rotating the connecting portion.

Preferably, the tumor screening system may further comprise lid open units, each comprising a moving portion approaching a position between the corresponding solution vial operating unit and the corresponding suction unit, and a rotating device installed on the moving portion, connected to a lid installed on the corresponding solution vial, and rotated.

The piston in the solution vial may comprise a piston rod having grooves formed therein and snap-connected to protrusions formed on two opposite flexible sheets of the solution vial so that the piston is fixed to a designated position of the solution vial, and the tumor screening system may further comprise piston connection releasing units, each comprising tapered protrusions, so that the two flexible sheets are inserted into a space between the protrusions, and fixed to one side of the system, a cylinder installed above the protrusions so that the cylinder is separated from the protrusions by a designated interval, and a cylinder rod inserted into and taken out of the cylinder.

### [Advantageous Effects]

The tumor screening system of the present invention operates separation of cells from a vial containing a solution mixed with the cells and collection of the cells on a slide using an automatic unit.

A collection vial serves to simultaneously store a solution containing cells and pour the solution out of the tumor screening system.

A brush uniformly collects cells from various regions of a woman's cervix, such as an inner wall and a transvaginal portion of cervix, without damage to the inner wall of a woman's vagina. Further, the brush allows the collected cells to be safely stored in a designated vial, thereby assuring stable and accurate cell collection and diagnosis.

### [Description of Drawings]

The above and other objects, features and other advantages of the present invention will be more clearly understood from the following detailed description taken in conjunction with the accompanying drawings, in which:
FIG. 1 is a perspective view of an upper portion of a filter used in a tumor screening system in accordance with the present invention;
FIG. 2 is a schematic perspective view illustrating the structure of the filter used in the tumor screening system in accordance with the present invention;
FIG. 3 is a perspective view of a lower portion of the filter used in the tumor screening system in accordance with the present invention;
FIG. 4 is a perspective view of a two row-type tumor screening system in accordance with the present invention;
FIG. 5 is a schematic side view of a mixed solution forming unit of the tumor screening system in accordance with the present invention;
FIG. 6 is a partially enlarged view of the tumor screening system of FIG. 5;
FIG. 7 is a partial sectional view illustrating the connection of a piston of a solution vial and an end of a cylinder rod of a piston operating unit of the tumor screening system in accordance with the present invention;
FIG. 8 is a schematic longitudinal sectional view of a lid open unit of the tumor screening system in accordance with the present invention;
FIG. 9 is a partial side view of a slide operating unit of the tumor screening system in accordance with the present invention;
FIG. 10 is a schematic sectional view of a suction unit of the tumor screening system in accordance with the present invention:
FIG. 11 is a schematic front view illustrating the connection of the solution vial to a piston connection releasing unit of the tumor screening system in accordance with the present invention;
FIG. 12 is a schematic side view illustrating the connection of the solution vial to the piston connection releasing unit of the tumor screening system in accordance with the present invention;
FIG. 13 is a schematic view partially illustrating a conventional solution vial and a conventional tumor screening system;
FIG. 14 is a schematic view illustrating conventional cytodiagnosis.

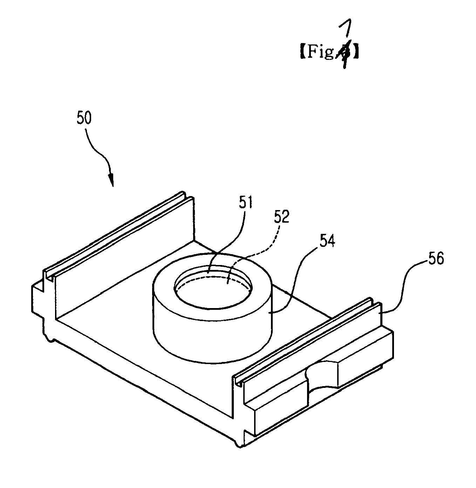

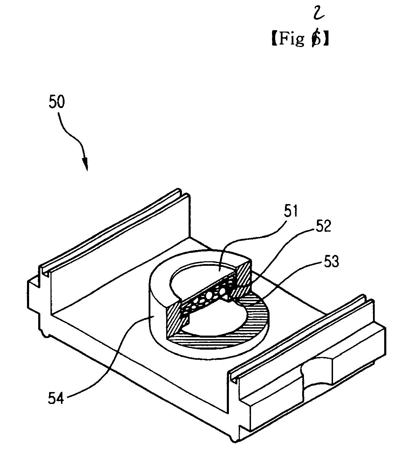

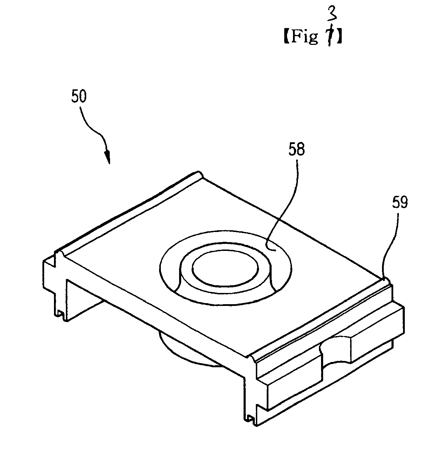

FIG. 1 is a perspective view of an upper portion of a filter used in a tumor screening system in accordance with the present invention, FIG. 2 is a schematic perspective view illustrating the structure of the filter used in the tumor screening system in accordance with the present invention, and FIG. 3 is a perspective view of a lower portion of the filter used in the tumor screening system in accordance with the present invention.

As shown in FIGS. 1 to 3, the filter 50 comprises a supporter 53 for supporting a cylindrical member 52, which has a coin-shaped cross section and air/water permeability and supports a collection film 51 having a laminated structure and provided with pores formed therethrough, a cylindrical connection flange 54 installed on the upper surface of the filter 50 around the supporter 53 and inserted into a space between the outer wall 34 and the inner connection wall 35 of the gauze film installation unit 30, upper guide members 56 having a designated height rectilinearly protruded from the upper surface of the filter 50 at both sides of the connection flange 54 so that a plurality of filters 50 can be vertically stacked and the stacked filters 50 slide against each other, and rectilinear grooves respectively formed in upper ends of the upper guide members 56 in the lengthwise direction of the upper guide members 56. A cylindrical connection groove 29 is formed in the center of the lower surface of the filter 50 so that a cylindrical protruding flange of the tumor screening system can be inserted into the cylindrical connection groove 29, and lower guide members 59 inserted into the rectilinear grooves of the upper guide members 56 of another filter 50 are formed at both sides of the cylindrical connection groove 29.

Thereafter, a tumor screening system using the solution vial of the present invention will be descried with reference to the annexed drawings.

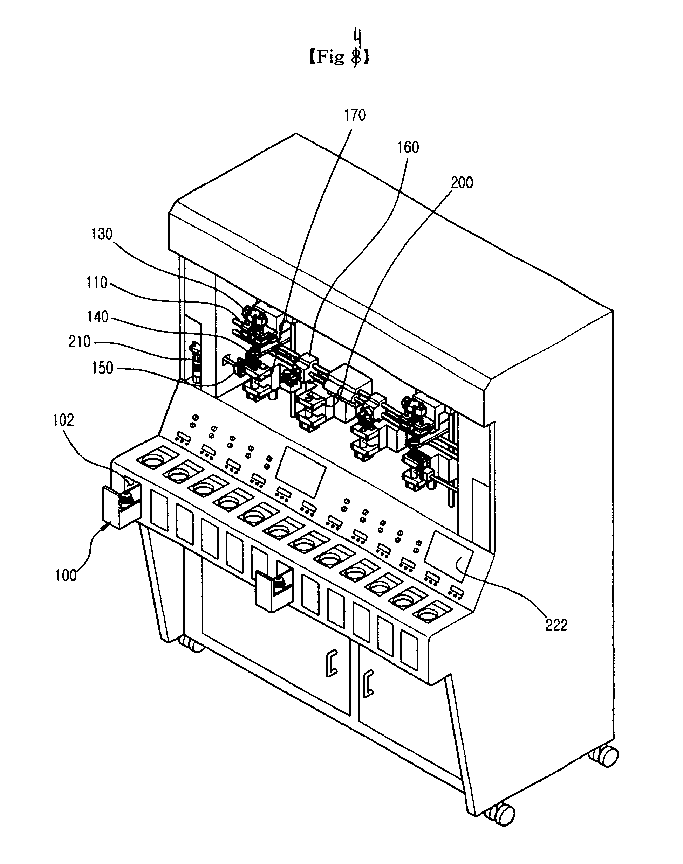

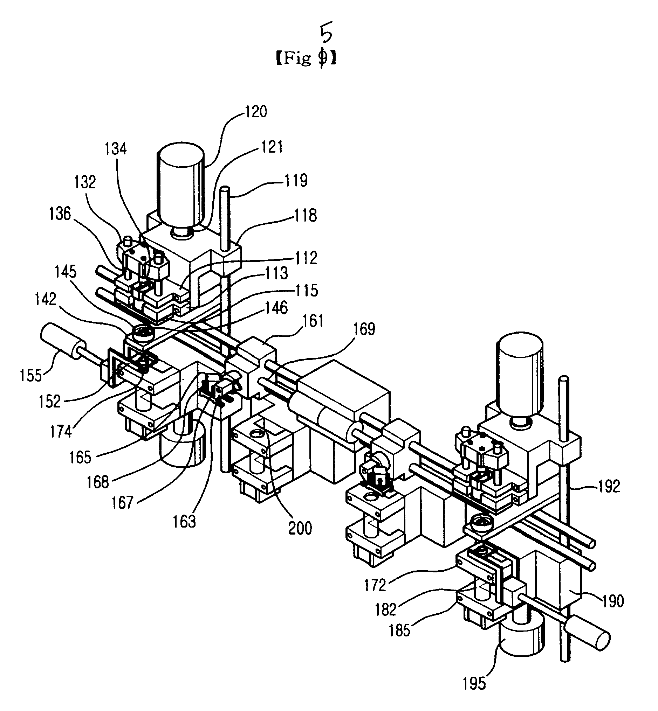

FIG. 4 is a perspective view of a two row-type tumor screening system in accordance with the present invention, and FIG. 5 is a partially enlarged view of the tumor screening system of FIG. 4. As shown in FIGS. 4 and 5, the tumor screening system comprises mixed solution forming units 100 for uniformly mixing cells with a solution filling the solution vials by rotating solution vials, solution vial operating units 110 for operating the solution vial, piston operating units 130 for operating the piston 20 installed in the solution vial, lid open units 140 for automatically opening and closing the lid 40 of the solution vial, filter operating units 150 for operating the filter 50, slide operating units 160 for operating a slide so that the slide contacts the filter 50, suction units 170 for sucking the mixed solution from the solution vial, alcohol receiving units 200 for receiving alcohol in which the slide is soaked, and piston connection releasing units 210 for separating the piston 20 from the solution vial.

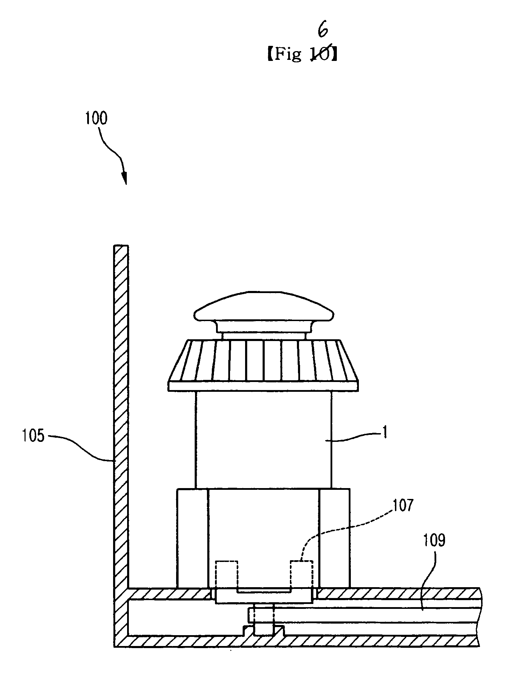

FIG. 6 is a schematic side view of the mixed solution forming unit of the tumor screening system in accordance with the present invention.

As shown in FIGS. 4 and 6, each of the mixed solution forming units 100 comprises a drawer-type receiving portion 105 inserted into and taken out of an agitating chamber 102 and having a space for receiving the solution vial, and a rotator 107 having protrusions connected to the casing 1 of the solution vial and connected to a motor (not shown) by a chain 109. The mixing of cells with the solution of the solution vial by the mixed solution forming unit 100 is achieved by rotating the solution vial using the rotator 107, after the receiving portion 105 is taken out of the agitating chamber 102, the solution vial is connected to the rotator 107, and then the receiving portion 105 is returned to the inside of the agitating chamber 102.

As shown in FIGS. 4 and 5, each of the solution vial operating units 110 comprises two-step blocks 112 and 113 vertically arranged in parallel for respectively supporting portions of the casing 1 of the solution vial having different outer diameters, the lowermost block 115 fixed to the lower block 113 of the two-step blocks 112 and 113 by a spring (not shown), and a moving block 118, to which the two-step blocks 112 and 113 are fixed, moving along a linear guide 119 for moving the two-step blocks 112 and 113. One side of the moving block 118 is fixed to a cylinder rod 121 of a fixed pneumatic cylinder 120. The lowermost block 115 has a hollow structure. The inner diameter of the lowermost block 115 is smaller than the outer diameter of the gauze film installation unit 30 so that the lowermost block 115 catches the gauze film installation unit 30, so as not to release the solution vial from the solution vial operating unit 110, and restricts the rotation of the gauze film installation unit 30.

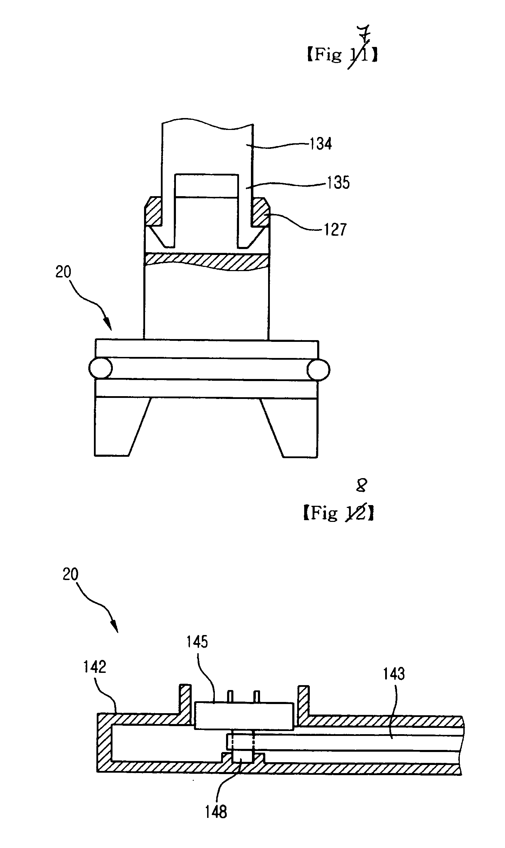

Each of the piston operating units 130 comprises a pneumatic cylinder 132 disposed above the two-step blocks 112 and 113 of each of the solution vial operating units 110 and fixed to the two-step blocks 112 and 113 through connection rods 136, and a cylinder rod 134 connected to the pneumatic cylinder 132 such that the cylinder rod 134 can rectilinearly move. The lower end of the cylinder rod 134 is connected to the rod portion 27 of the piston 20 of the solution vial. As shown in FIG. 7, a pair of branched hooks 135 are formed on the lower end of the cylinder rod portion 27, and are snap-connected to the through hole of the rod portion 27 of the piston 20. The cylinder rod 134 of the piston operating unit 130 is made of synthetic resin, thus having flexibility.

The blocks 112 and 113 of the solution vial operating unit 110 and the pneumatic cylinder 132 of the piston operating unit 130 are integrally fixed to a pair of the connection rods 136, which are formed through both sides thereof. The lowermost block 115 of the solution vial operating unit 110 is elastically fixed to the lower block 113 of the two-step blocks 112 and 113 by the spring (not shown).

FIG. 8 is a schematic longitudinal sectional view of the lid open unit 140 of the tumor screening system of the present invention. As shown in FIGS. 4, 5, and 8, each of the lid open units 140 comprises a moving portion 142 installed below the solution vial operating unit 110 and moving back and forth, and a lid rotator 145 installed on the upper surface of the moving portion 142. The lid rotator 145 has a depressed structure so that the lid 40 is laid on the upper surface of the lid rotator 145, three connection protrusions 146, which are inserted into the connector insertion holes 42 of the lid 40, are formed on the center of the lid rotator 145, and a rotary shaft 148 extended downwardly from the lid rotator 145 is contained in the moving portion 142 and connected to a chain 143 driven by a motor.

As shown in FIG. 5, each of the filter operating units 150 comprises a pneumatic cylinder 155, and clamps 152 moved by the pneumatic cylinder 155 for clamping the filter 50. The clamps 152 clamp the filter 50 picked up from a filter loader having a plurality of the filters 50 loaded therein, and carry the filter 50 to a standby position.

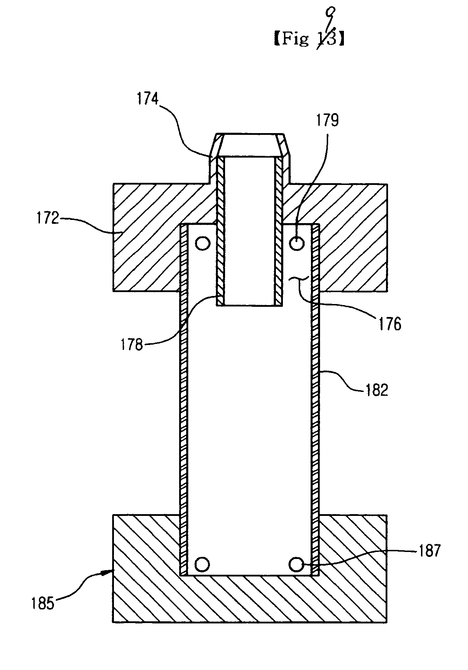

FIG. 9 is a partial side view of the slide operating unit of the tumor screening system of the present invention.

As shown in FIGS. 4, 5, and 9, the slide operating units 160 move between the filter standby position and a slide drop position to cause contact between the slide and the filter 50, and drops the slide contacting the filter 50 into a solution containing coagulated cells. Each of the slide operating units 160 comprises a moving portion 161 connected to a cylinder rod 169 operated by a pneumatic cylinder 165, a rotating portion 163 rotatably hinged to the moving portion 161, and a slide mounting portion 167 elastically fixed to the lower part of the rotating portion 163. The moving portion 161 moves along the linear guide 119, the rotating portion 163 is provided with the pneumatic cylinder 165, and the slide mounting portion 167 is connected to the cylinder rod 169 connected to the pneumatic cylinder 165. The rotating portion 163 includes a rectangular plane extended from the hinged portion thereof, and is connected to a motor, thus being rotatable in a designated angle range. The slide mounting portion 167 is elastically fixed to the rectangular plane via bolts 168, each being provided with a spring. A guide groove is formed in the lower surface of the slide mounting portion 167 so that the slide can be inserted into the guide groove.

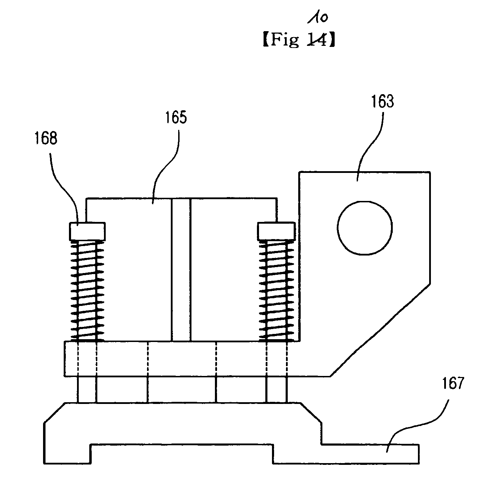

FIG. 10 is a schematic sectional view of the suction unit of the tumor screening system of the present invention.

As shown in FIGS. 4, 5, and 10, the suction units 170 suck the mixed solution in the solution vial via the filter 50. Each of the suction unit 170 comprises a filter connecting portion 172 having a hollow structure and connected to the lower portion of the filter 50 under the collection film 51, a solution receiving tube 182 connected to the lower portion of the hollow of the filter connecting portion 172 for guiding the solution downwardly, a solution receiving tube supporter 185 connected to the lower end of the light receiving tube 182 and having solution discharge holes 187 for discharging the solution, and a moving block to which the filter connecting portion 172 and the solution receiving tube supporter 185 are fixed. The moving block is connected to a pneumatic cylinder unit so that the moving block can move along the linear guide 119. An interference fit portion 174 connected to the cylindrical connection groove 58 of the filter 50 is formed on the upper surface of the filter connecting portion 172, an inner space 176, the lower end surface of which is opened, is formed in the filter connecting portion 172, and a solution guide nozzle 178 is connected to the lower portion of the interference fit portion 174 and is extended downwardly via the inner space 176. The inner space 176 of the filter connecting portion 172 is connected to suction holes 179 for sucking air in the inner space 176. The suction holes 179 are connected to a sucking instrument (not shown) having a similar structure to that of an injector. The solution receiving tube supporter 185 has an inner space connected to the solution receiving tube 182, and the solution discharge holes 187, which are selectively opened and closed to discharge the solution to the outside, are formed through one side of the inner space. The solution discharge holes 187 may discharge the solution to a reservoir installed in the system.

As shown in FIG. 5, each of the alcohol receiving units 200 receives alcohol for coagulate cells of the slide, and the upper portion of each of the alcohol receiving units 200 is opened so that the dropped slide is dipped into the alcohol therethrough.

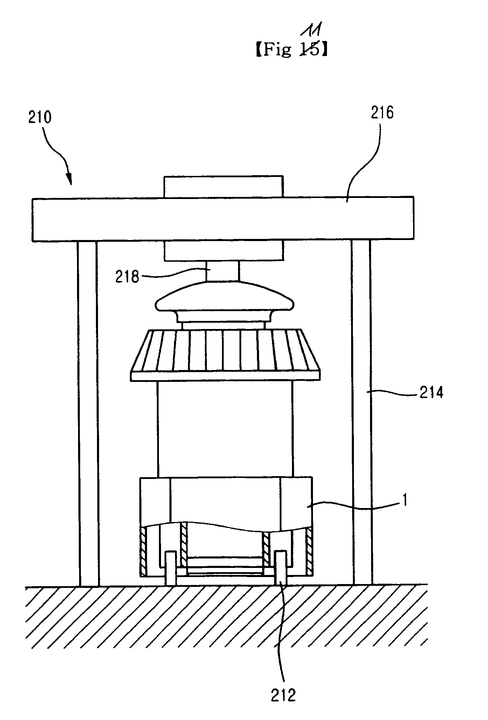

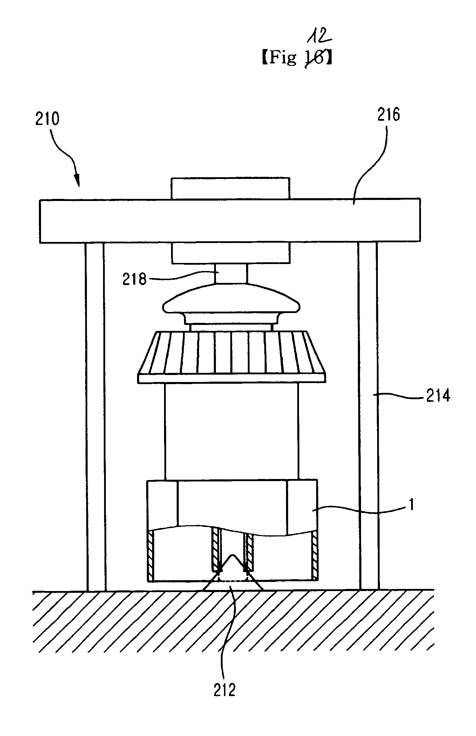

FIG. 11 is a schematic front view illustrating the connection of the solution vial to the piston connection releasing unit of the tumor screening system in accordance with the present invention, and FIG. 12 is a schematic side view illustrating the connection of the solution vial to the piston connection releasing unit of the tumor screening system in accordance with the present invention.

As shown in FIGS. 11 and 12, the piston connection releasing units 210 release the snap connection of the guide slit 8 of the solution vial and the rod portion 27 of the piston 20. Each of the piston connection releasing units 210 comprises a pair of triangular protrusions 212 formed on a table, and a pneumatic cylinder unit installed above the triangular protrusions 212 through connection rods 214. Lower portions of the triangular protrusions 212 have a width larger than the interval between the first planes 9 of the guide slit 8. The pneumatic cylinder unit comprises a pneumatic cylinder 216 fixed to the connection rods 214, and a cylinder rod 218 pressing the solution vial.

The tumor screening system of the present invention further comprises LCD panels 222 for displaying the operations of the above pneumatic cylinders and the positions of the moving members according to the operations.

Hereinafter, a process for mixing cells with a solution using a solution vial and transferring the cells from the solution vial to a slide by the tumor screening system of the present invention will be described.

First, a step of putting a cell collected member into the solution vial in order to mix collected cells with the solution is performed.

A user separates the gauze film installation unit 30, to which the lid 40 is connected, from the casing 1 by turning the gauze film installation unit 30 by hand, and injects a solution for soaking cells into the casing 1. Thereafter, the user puts the cell collected member into the casing 1, and connects the gauze film installation unit 30 to the casing 1.

Second, a step of installing the solution vial having the cell collected member and transferring the collected cells from the solution vial to a slide is performed.

The receiving portion 105 of the mixed solution forming unit 100 is opened, and the solution vial having the cell collected member is installed in the receiving portion 105. More specifically, the lower portion of the casing 1 is connected to protrusions of a rotating plate formed in the receiving portion 105, and the receiving portion 105 having the solution vial is pushed into the agitating chamber 102. The rotating plate is rotated at a predetermined speed for a predetermined time using a control unit. When the solution vial is rotated, the agitating protrusions 5 of the solution agitating portion 4 of the casing 1 and the impellers 24 of the piston 20 stir the solution in the solution vial, and the cells are separated from the cell collected member and are mixed with the solution in the solution vial.

The solution vial, in which the cells and the solution are sufficiently mixed with each other, is inserted into a space between the two-step blocks 112 and 113 under the condition that the lowermost block 115 of the solution vial operating unit 110 is lowered to a designated distance by pressing, and, when the force pressing the lowermost block 115 is eliminated, the lowermost block 115 is elevated by the restoring force of the spring, and the lowermost block 115 pushes the solution vial upwardly under the condition that the gauze film installation unit 30 of the solution vial is caught by the hollow of the lowermost block 115, thus fixing the solution vial to the solution vial operating unit 110. When the pneumatic cylinder 132 of the piston operating unit 130 is operated so that the cylinder rod 134 is lowered under the above condition, the branched hooks 135 formed on the lower end of the cylinder rod portion 27 are snap-connected to the through hole of the rod portion 27 of the piston 20. Accordingly, the connection between the piston 20 of the solution vial and the piston operating unit 130 under the condition that the solution vial is fixed to the solution vial operating unit 110 is completed.

Thereafter, the clamps of the filter operating unit 150 clamp the filter 50 slid out of the filter loader, transfers it to a position above the suction unit 170, and stops.

Thereafter, when the moving portion 142 of the lid open unit 140 moves forwardly, the lid rotator 145 is located below the lid 40 of the solution vial. Then, when the pneumatic cylinder 120 of the solution vial operating unit 110 is operated so that the moving block 118 is lowered, the two-step blocks 112 and 113 integrally fixed to the moving block 118 are lowered and the solution vial is lowered also. Here, the protrusions 146 of the lid rotator 145 are inserted into the connector insertion holes 42 of the lid 40 of the solution vial, and when the motor is controlled so that the lid rotator 145 is rotated, the screw connection between the lid 40 and the gauze film installation unit 30 is released so that the lid 40 is separated from the gauze film installation unit 30, and the pneumatic cylinder 120 is operated so that the moving block 118 of the solution vial operating unit 110 is elevated to a distance corresponding to the height of the lid 40, thereby allowing the lid 40 to be smoothly opened. When the pneumatic cylinder 132 of the piston operating unit 130 is operated together with the opening of the lid 40 so that the cylinder rod 134 is elevated, the piston 20 connected to the end of the cylinder rod 134 is elevated to lower the internal pressure of the casing 1 of the solution vial, thereby preventing the solution from pouring out of the solution vial. The moving portion 142 of the lid open unit 140, on which the separated lid 40 is laid, moves backwardly.

When the pneumatic cylinder 120 of the solution vial operating unit 110 is operated under the condition that the lid 40 of the solution vial is opened, the moving block 118 is lowered, and the connection flange 54 on the upper surface of the filter 50 is connected to a space between the outer wall 34 and the inner connection wall 35 of the gauze film installation unit 30 by interference fit, and simultaneously, when the pneumatic cylinder of the suction unit 170 is operated so that the moving block of the suction unit 170 is elevated, the interference fit portion 174 on the upper surface of the filter connecting portion 172 is connected to the cylindrical connection groove 58 formed in the lower surface of the filter 50 by interference fit. Accordingly, the solution vial, the filter 50, and the suction unit 170 are connected in a hermetically sealed state. Since the filter 50 is supported by the clamps of the filter operating unit 150, the solution vial and the suction unit 170 are simultaneously connected to the filter 50.

Thereafter, when the sucking instrument connected to the filter connecting portion 172 is operated under the condition that the solution discharge holes 187 formed through the solution receiving tube supporter 185 of the suction unit 170 are closed, the pressure in the filter connecting portion 172 and the pressure in the solution receiving tube 182 are lowered, and the mixed solution is sucked through the gauze film 32 and is filtered by the collection film 51 of the filter 50. The solution without cells flows toward the solution receiving tube 182. The amount of the mixed solution sucked from the solution vial is adjusted by controlling the sucking instrument. When the solution is discharged from the solution vial, the pneumatic cylinder 132 of the piston operating unit 130 is not operated and the piston 20 freely descends.

Thereafter, when the pneumatic cylinder 120 of the solution vial operating unit 110 is operated to elevate the moving block 118 and the pneumatic cylinder 132 of the piston operating unit 130 is operated to elevate the piston 20 of the solution vial, the solution vial is located on the filter 50 and the pressure in the casing 1 is not lowered, thereby preventing the mixed solution in the solution vial from pouring out downward.

Thereafter, the lid open unit 140 moves forwardly again under the condition that the solution vial is elevated, and a process opposite to the process for opening the lid 40 is performed, thus closing the solution vial with the lid 40.

When the pneumatic cylinder 132 of the piston operating unit 130 is operated to elevate the cylinder rod 134 to be distant from the solution vial closed with the lid 40, the piston 20 caught by the end of the cylinder rod 134 is elevated, and the connection grooves 29 of the rod portion 27 of the piston 20 are snap-connected to the fixing protrusions 10 of the guide slit 8 of the casing 1. Since the contact portion 22 of the piston 20 contacts the upper portion of the casing 1 under the snap-connected state, when the cylinder rod 134 is continuously elevated, the end of the cylinder rod 134 is slidably separated from the rod portion 27 of the piston 20. Since the piston 20 is forcibly drawn from the solution vial closed with the lid 40, the inside of the casing 1 becomes a vacuum state.

Thereafter, the solution vial is taken out of the solution vial operating unit 110 under the condition that the lowermost block 115 of the solution vial operating unit 110 is pressed downwardly.

Thereafter, when the sucking instrument of the suction unit 170 is additionally operated under the condition that the solution vial is separated from the filter 50, the solution remaining on the collection film 51 of the filter 50 is sucked into the lower portion of the system.

Thereafter, the pneumatic cylinder 165 connected to the moving portion 161 is operated to move the slide operating unit 160 on the filter 50 and the pneumatic cylinder connected to the slide mounting portion 167 is operated to lower the slide mounting portion 167 to the filter 50, thereby causing a slide to contact the collection film 51 of the filter 50. When the moving portion 161 is horizontally moved under the condition that the slide contacts the filter 50, the slide repeatedly contacts the collection film 51.

The slide receives cells from the collection film 51 of the filter 50, and is transferred to be above the alcohol receiving unit 200. Then, when the rotating portion 163 is rotated at an angle of 90 , the linear guide groove is perpendicularly erected and the slide is separated from the guide groove and dropped into the alcohol receiving unit 200.

The slide, which is soaked in alcohol, is taken out of the alcohol receiving unit 200, and is used to test the cells thereon.

After testing, the solution vial, which is stored under the condition that the inside of the casing 1 is in the vacuum state, may be used again. In this case, the piston connection releasing unit 210 releases the snap-connection between the piston 20 and the casing 1. More specifically, when the pneumatic cylinder 216 is operated under the condition that the solution vial is located at a position so that the triangular protrusions 212 of the piston connection releasing unit 210 are inserted into a space between the first planes 9 having the fixing protrusions 10 of the guide slit 8 of the solution vial, and the solution vial is pressed downwardly, the space between the first planes 9 is widened by the triangular protrusions 212, thereby releasing the snap-connection between the piston 20 and the casing 1 and allowing the piston 20 to descend to a position corresponding to the amount of the remaining solution in the solution vial. That is, the piston 20 is returned to a state freely moving against the solution vial.

The present invention is applied to a tumor screening system.

Although the preferred embodiments of the present invention have been disclosed for illustrative purposes, those skilled in the art will appreciate that various modifications, additions and substitutions are possible.

## Claims

1. A tumor screening system comprising:
solution vial operating units (110), each comprising a fixing unit for fixing a solution vial having a piston (20) installed therein, and a cylinder device for linearly moving the solution vial toward a filter connected to the solution vial;
piston (20) operating units, each comprising a cylinder fixed to the corresponding solution vial operating unit, and a cylinder rod installed on the cylinder and fixed to one portion of the piston (20) of the solution vial for linearly moving piston;
suction units (170), each comprising a connecting portion (174) disposed under the corresponding solution vial operating unit and connected to the filter (50), and a tube connected to the connecting portion for guiding a solution filling the solution vial, and a suction device connected to the inner space of the tube; and
slide operating units (160), each comprising a slide mounting portion (161) on which a slide is mounted, and a moving device for moving the slide mounting portion to achieve contact between the slide and the filter connected to the corresponding suction unit (170).

2. A tumor screening system as claimed in claim 1, further comprising alcohol receiving units (200) for receiving alcohol,
wherein each of the slide operating units (160) further comprises a slide dropping unit for dropping the slide into the corresponding alcohol receiving unit.

3. A tumor screening system as claimed in claim 1, further comprising filter operating units (150), each comprising a moving unit for moving the filter to a position under the solution vial, and a fixing unit for fixing the filter.

4. A tumor screening system as claimed in claim 1, further comprising mixed solution forming units (100), each comprising a connecting portion, to which the solution vial is connected, and a rotating portion for rotating the connecting portion.

5. A tumor screening system as claimed in claim 1, further comprising lid open units (140), each comprising a moving portion (142) approaching a position between the corresponding solution vial operating unit and the corresponding suction unit, and a rotating device installed on the moving portion, connected to a lid installed on the corresponding solution vial, and rotated.

6. A tumor screening system as claimed in claim 1, wherein the piston in the solution vial comprises a piston rod having grooves formed therein and snap-connected to protrusions formed on two opposite flexible sheets of the solution vial so that the piston (20) is fixed to a designated position of the solution vial,
further comprising piston connection releasing units (210), each comprising tapered protrusions, so that the two flexible sheets are inserted into a space between the protrusions, and fixed to one side of the system, a cylinder installed above the protrusions so that the cylinder is separated from the protrusions by a designated interval, and a cylinder rod inserted into and taken out of the cylinder.

## Patentansprüche

1. A tumor-Screening-System, umfassend:
Lösungsfläschchen handhabende Einheiten (110), die jeweils eine Fixiereinheit für das Fixieren eines Lösungsfläschchens, das einen darin eingebauten Kolben (20) aufweist, und eine Zylindervorrichtung für das lineare Bewegen des Lösungsfläschchens auf einen Filter zu, der mit dem Lösungsfläschchen verbunden ist, umfassen;
Kolben (20) handhabende Einheiten, die jeweils einen an die entsprechende Lösungsfläschchen handhabende Einheit befestigten Zylinder und einen an dem Zylinder montierten und an einem Teil des Kolbens (20) des Lösungsfläschchens befestigten Zylinderstab für das lineare Bewegen des Kolbens umfassen;
Saugeinheiten (170), die jeweils einen unter der entsprechenden Lösungsfläschchen handhabenden Einheit angeordneten und mit dem Filter (50) Verbundenen Verbindungsteil (174) und ein mit dem Verbindungsteil verbundenes Röhrchen für das Leiten einer das Lösungsfläschchen füllenden Lösung und eine mit dem inneren Raum des Röhrchens verbundene Saugvorrichtung umfassen; und
Objektträger handhabende Einheiten (160), die jeweils einen Objektträgerbefestigungsteil (161), auf dem ein Objektträger befestigt wird, und eine Bewegungsvorrichtung für das Bewegen des Objektträgerbefestigungsteils, um Kontakt zwischen dem Objektträger und dem mit der entsprechenden Saugeinheit (170) verbundenen Filter zu erreichen, umfassen.

2. Tumor-Screening-System wie i n Anspruch 1 beansprucht, weiter umfassend Alkohol aufnehmende Einheiten (200) für die Aufnahme von Alkohol,
wobei jede der Objektträger handhabenden Einheiten (160) weiter eine Objektträger abwerfende Einheit für das Abwerfen des Objektträgers in die entsprechende Alkohol aufnehmende Einheit umfasst.

3. Tumor-Screening-System wie i n Anspruch 1 beansprucht, weiter Filter handhabende Einheiten (150) umfassend, die jeweils eine Bewegungseinheit für das Bewegen des Filters in eine Position unterhalb des Lösungsfläschchens und eine Fixiereinheit für das Fixieren des Filters, umfassen.

4. Tumor-Screening-System wie in Anspruch 1 beansprucht, weiter Lösungsgemische bildende Einheiten (100) umfassend, die jeweils einen Verbindungsteil, mit dem das Lösungsfläschchen verbunden wird, und einen sich drehenden Teil für das Drehen des Verbindungsteils umfassen.

5. Tumor-Screening-System wie in Anspruch 1 beansprucht, weiter Deckel öffnende Einheiten (140) umfassend, die jeweils einen sich bewegenden Teil (142), der sich einer Position zwischen der entsprechenden Lösungsfläschchen handhabenden Einheit und der entsprechenden Saugeinheit nähert, und eine auf dem sich bewegenden Teil montierte Drehvorrichtung, die mit einem auf dem entsprechenden Lösungsfläschchen montierten Deckel verbunden ist und gedreht wird, umfassen.

6. Tumor-Screening-System wie in Anspruch 1 beansprucht, wobei der Kolben in dem Lösungsfläschchen einen Kolbenstab umfasst, der darin gebildete Kerben aufweist und über eine Schnappverbindung mit Vorsprüngen verbunden ist, die an zwei sich gegenüberliegenden flexiblen Scheiben des Lösungsfläschchens gebildet sind, so dass der Kolben (20) in einer vorbestimmten Position des Lösungsfläschchens fixiert ist,
weiter umfassend die Kolbenverbindung lösende Einheiten (210), die jeweils spitz zulaufende Vorsprünge umfassen, so dass die zwei flexiblen Scheiben in einen Raum zwischen den Vorsprüngen eingeführt und auf einer Seite des Systems fixiert werden, wobei ein Zylinder oberhalb der Vorsprünge montiert ist, so dass der Zylinder von den Vorsprüngen durch einen Vorbestimmten Zwischenraum getrennt ist und ein Zylinderstab in den Zylinder eingeführt und daraus herausgenommen wird.

## Revendications

1. Système de dépistage de tumeur comprenant: des unités de contrôle de flacon de solution (110), comprenant chacune une unité de fixation pour fixer un flacon de solution ayant un piston (20) installé à l'intérieur, et un dispositif à cylindre pour déplacer linéairement le flacon de solution vers un filtre raccordé au flacon de solution;
des unités de contrôle de piston (20), comprenant chacune un cylindre fixé à l'unité de contrôle de flacon de solution correspondante, et une tige de cylindre installée sur le cylindre et fixée à une partie du piston (20) du flacon de solution, pour déplacer linéairement le piston;
des unités d'aspiration (170), comprenant chacune une partie de raccordement (174) disposée sous l'unité de contrôle de flacon de solution correspondante et connectée au filtre (50), et un tube raccordé à la partie de raccordement, pour guider une solution remplissant le flacon de solution, et un dispositif d'aspiration raccordé à l'espace interne du tube; et
des unités de contrôle de lame (160) comprenant chacune une partie de montage de lame (161), sur laquelle une lame est montée et un dispositif de déplacement pour déplacer la partie de montage de lame pour obtenir un contact entre la lame et le filtre raccordé à l'unité d'aspiration correspondante (170).

2. Système de dépistage de tumeur selon la revendication 1, comprenant en outre des unités de réception d'alcool (200) pour recevoir de l'alcool, dans lequel chacune des unités de contrôle de lame (160) comprend en outre une unité de délivrance de lame pour laisser tomber la lame dans l'unité de réception d'alcool correspondante.

3. Système de dépistage de tumeur selon la revendication 1, comprenant en outre des unités de contrôle de filtre (150), comprenant chacune une unité de déplacement pour déplacer le filtre à une position sous le flacon de solution, et une unité de fixation pour fixer le filtre.

4. Système de dépistage de tumeur selon la revendication 1, comprenant en outre des unités de formation de solution mixtes (100), comprenant chacune une partie de raccordement, à laquelle le flacon de solution est raccordé, et une partie rotative pour entraîner en rotation la partie de raccordement.

5. Système de dépistage de tumeur selon la revendication 1, comprenant en outre des unités d'ouverture de couvercle (140), comprenant chacune une partie mobile (142) approchant d'une position entre l'unité de contrôle de flacon de solution correspondante et l'unité d'aspiration correspondante, et un dispositif rotatif installé sur la partie mobile, raccordé à un couvercle installé sur le flacon de solution correspondant, et entraîné en rotation.

6. Système de dépistage de tumeur selon la revendication 1, dans lequel le piston dans le flacon de solution comprend une tige de piston ayant des cannelures formées à l'intérieur et connectées par des griffes à des saillies formées sur deux feuilles flexibles opposées du flacon de solution, de sorte que le piston (20) soit fixé dans une position désignée du flacon de solution, comprenant en outre les unités de libération de raccordement du piston (210), chacune comprenant des saillies coniques, de sorte que les deux feuilles flexibles soient insérées dans un espace entre les saillies, et fixées à un côté du système, un cylindre installé au-dessus des saillies de sorte que le cylindre soit séparé des saillies par un intervalle désigné, et une tige de cylindre insérée dans et extraite du cylindre.
